# EUROPEAN PATENT SPECIFICATION

(11) **EP 1 269 845 B1**
(45) Date of publication and mention of the grant of the patent: **22.03.2006**
(21) Application number: 01901471.1
(22) Date of filing: 19.01.2001
(51) Int. Cl.: A01N 43/08, A61K 47/10, A61K 47/12, A61K 47/14, A23L 3/3562, C07H 3/10, A61K 31/7004, A23L 1/16, A23L 1/212, A23L 1/216, A23L 3/3472, A23L 3/3508, A61K 31/7008

(54) **BACTERIAL GROWTH REGULATORS OR INHIBITORS WITH THE USE OF 1,5-D-ANHYDROFRUCTOSE**
1,5-D-ANHYDROFRUCTOSE ENTHALTENDE REGULATOREN ODER INHIBITOREN VON BAKTERIELLEM WACHSTUM
REGULATEURS OU INHIBITEURS DE CROISSANCE BACTERIENNE CONTENANT 1,5-D-ANHYDROFRUCTOSE

(30) Priority: 28.03.2000 JP 2000087905; 30.03.2000 JP 2000094791
(43) Date of publication of application: 02.01.2003
(73) Proprietor: Nihon Starch Co., Ltd., Kagoshima-shi, Kagoshima 891-0122 (JP); Hizukuri, Tomiko, Kagoshima-shi, Kagoshima (JP); Hizukuri, Kazuko, Kagoshima-shi Kagoshima (JP); Hizukuri, Kaoru, Chiba-shi, Chiba (JP); Abe, Junichi, Kagoshima-shi, Kagoshima 891-0145 (JP)
(72) Inventor: HIZUKURI, Susumu, Kagoshima-shi Kagoshima 892-0811 (JP); ABE, Junichi, Kagoshima-shi Kagoshima 891-0145 (JP); TAKEDA, Yasuhito, Hioki-gun Kagoshima 899-2704 (JP); MUROYA, Kenkou, Kagoshima-shi Kagoshima 891-0122 (JP); YOSHINAGA, Kazuhiro, Kagoshima-shi Kagoshima 891-0122 (JP); FUJISUE, Mami, Kagoshima-shi Kagoshima 891-0122 (JP); ISHIBA, Hideto, Kagoshima-shi Kagoshima 891-0122 (JP)
(74) Representative: Cresswell, Thomas Anthony
(86) International application number: PCT/JP2001/000354
(87) International publication number: WO 2001/072124

(56) References cited:
- EP-A1- 0 384 319
- WO-A1-89/12399
- WO-A2-95/10616
- JP-A- 7 039 355
- JP-A- 7 039 356
- JP-A- 54 122 796
- FUJISUE M ET AL: "PREPARATION AND ANTIOXIDATIVE ACTIVITY OF 1,5-ANHYDROFRUCTOSE" OYO TOSHITSU KAGAKU - JOURNAL OF APPLIED GLYCOSCIENCE, NIHON OYO TOSHITSU KAGAKUKAI, TSUKUBA, JP, vol. 46, no. 4, 1999, pages 439-444, XP002937467 ISSN: 1340-3494
- MURATA A ET AL: "KILLING EFFECT OF ASCORBIC ACID ON BACTERIA AND YEASTS" BITAMIN - VITAMINS, JOURNAL OF THE VITAMIN SOCIETY OF JAPAN, KYOTO, JP, vol. 64, no. 12, 1990, pages 709-713, XP002937468 ISSN: 0006-386X
- BAUTE M.A. ET AL.: 'Fungal bioconversions yielding unusual antibiotic pyrones from sugars. XV. Biogenesis of 1,5-D-anhydrofructose, the precursor of microthecin in morels: a novel degradation of .alpha.-D-1,4-glucans such as glycogen or starch' BULL. SOC. PHARM. vol. 128, 1989, pages 9 - 18, XP002941402
- BAUTE M.A. ET AL.: 'Fungal enzymic activity degrading 1,4-alpha-D-glucans to 1,5-D-anhydrofructose' PHYTOCHEMISTRY vol. 27, no. 11, 1988, pages 3401 - 3403, XP002941403
- YU S. ET AL.: 'Methods of the assay of 1,5-D-anhydrofructose and .alpha.-D-1,4-glucan lyase' CARBOHYDRATE RESEARCH vol. 305, 1998, pages 73 - 82, XP002941404

## Description

### Technical Field

The present invention relates to an agent for suppressing or inhibiting growth of bacteria by use of 1,5-D-anhydrofructose, and use of the agent. More specifically, the present invention relates to an agent for suppressing or inhibiting growth of bacteria effectively by using 1,5-D-anhydrofructose in combination with an antioxidant and/or a chelating agent, and use of the agent.

### Background Art

1,5-D-anhydrofructose can be produced, using starch or decomposed starch as a substrate, by action of α-1,4-glucanlyase which is present in a microorganism such as basidiomycetes or plant tissue such as red algae. 1,5-D-anhydrofructose has an interesting, peculiar structure, dehydrated form of glucose. As for its functionality, it has been reported that it has an effect of suppressing or inhibiting growth of bacteria.

### Disclosure of the Invention

An object of the present invention is to provide use of 1,5-D-anhydrofructose for suppressing or inhibiting growth of bacteria in the co-presence of an antioxidant and/or a chelating agent, based on a newly found fact that 1,5-D-anhydrofructose exhibits an effect of suppressing or inhibiting growth of bacteria at a lower concentration when used in combination with an antioxidant and/or a chelating agent.

Another object of the present invention is to provide an agent for suppressing or inhibiting growth of bacteria which contains an antioxidant and/or a chelating agent and 1,5-D-anhydrofructose as active ingredients.

Other objects and advantages of the present invention will be apparent from the following description.

According to the present invention, firstly, the above objects and advantages of the present invention are achieved by an agent for suppressing or inhibiting growth of bacteria which comprises:
(A) 1,5-D-anhydrofructose, and
(B) at least one compound selected from the group consisting of an antioxidant and a chelating agent.

Further, according to the present invention, secondly, the above objects and advantages of the present invention are achieved by use of 1,5-D-anhydrofructose for suppressing or inhibiting growth of bacteria in the co-presence of at least one compound selected from the group consisting of an antioxidant and a chelating agent.

According to the present invention, in a case where a variety of bacteria are cultured in the co-presence of either an antioxidant or a chelating agent and 1,5-D-anhydrofructose, a significant enhancement of an effect of suppressing or inhibiting growth of bacteria is observed, as compared with a case where the bacteria are cultured in the presence of 1,5-D-anhydrofructose at the same concentration without any of the antioxidant or the chelating agent. Further, use of 1,5-D-anhydrofructose in combination with both the antioxidant and the chelating agent particularly enhances the effect.

To use the agent of the present invention, for example, directly incorporated into a product, for preserved, such as food, a beverage, a drug, cosmetic or detergent or can be included in packages of these products instead.

When a variety of bacteria are cultured in agar media in the presence of 1,5-D-anhydrofructose without any of an antioxidant or a chelating agent, a minimum growth inhibitory gram-negative bacteria and 1.0 to 3.0% for gram-positive bacteria. Under the circumstances, influences on growth of a variety of bacteria in the presence of 1, 5-D-anhydrofructose with or without sodium ascorbate which is a typical antioxidant to be used in food and beverages were examined. Strains of bacteria used in the this test as well as their providers are shown in Table 1.

**Table 1**

| | | | |
|---|---|---|---|
| Gram-Negative Bacteria | *Escherichia coli* | IFO | 3301 |
| | *Pseudomonas aeruginosa* | IFO | 12689 |
| | *Proteus vulgaris* | IFO | 3851 |
| | *Enterobactor cloacae* | JCM | 1232 |
| Gram-Positive Bacteria | *Bacillus subtillis* | IFO | 3009 |
| | *Bacillus cereus* | IFO | 3131 |
| | *Lactobacillus casei* | ATCC | 393 |
| | *Streptococcus eqinus* | ATCC | 9812 |

After the bacterial strains were suspended in physiological salt solutions, they were inoculated into agar media (each containing 0.25% of yeast extract, 0.5% of polypeptone, 1.0% of glucose and 1.5% of soft agar and having a pH of 6.5) with platinum loops and then cultured at 37°C until colonies were formed. Thereafter, the bacteria were inoculated, with sterilized toothpicks, from the colonies into agar media of the above composition each of which contained 1.0% of sodium ascorbate and 1.0% of 1,5-D-anhydrofructose, and into agar media of the above composition each of which contained 1.0% of 1,5-D-anhydrofructose and no sodium ascorbate. For controls, culture media of the same composition each of which contained neither sodium ascorbate nor 1,5-D-anhydrofructose and culture media of the same composition each of which contained 1.0% of sodium ascorbate and no 1,5-D-anhydrofructose were used. Abilities of sodium ascorbate and 1,5-D-anhydrofructose to suppress/inhibit growth of the bacteria were evaluated by visually observing the colonies formed after the bacteria were cultured at 37° C for one night. The results are shown in Table 2.

**Table 2**

| | 1 | 2 | 3 | 4 |
|---|---|---|---|---|
| *Escherichia coli* | + | + | + | ± |
| *Pseudomonas aeruginosa* | + | + | + | + |
| *Proteus vulgaris* | + | + | ± | - |
| *Enterobactor cloacae* | + | + | + | + |
| *Bacillus subtillis* | + | + | + | ± |
| *Bacillus cereus* | + | + | ± | ± |
| *Lactobacillus casei* | + | + | ± | - |
| *Streptococcus eqinus* | + | + | ± | - |

| | | | | |
|---|---|---|---|---|
| 1 ... Neither sodium ascorbate nor 1,5-D-anhydrofructose was added. | | | | |
| 2 ... 1% of sodium ascorbate was added. | | | | |
| 3 ... 1% of 1,5-D-anhydrofructose was added. | | | | |
| 4 ... 1% of sodium ascorbate and 1% of 1,5-D-anhydrofructose were added. | | | | |
| +, ± and - represent the following conditions of the colonies. | | | | |
| +: A colony when neither sodium ascorbate nor 1, 5-D-anhydrofructose is added and a colony which is as large as the colony when neither sodium ascorbate nor 1,5-D-anhydrofructose is added. | | | | |
| ±: A colony can be detected but its growth is apparently inhibited. | | | | |
| -: A colony cannot be detected. | | | | |

Table 2 shows the following. 1.0% of sodium ascorbate does not have a growth suppressing ability against any of the bacteria. Meanwhile, 1.0% of 1,5-D-anhydrofructose exhibits a growth inhibitory effect to some extent, but its growth inhibiting effect is further enhanced in the co-presence of 1.0% of sodium ascorbate. The same experiment was carried out by using sodium erythorbate which was an isomer of sodium ascorbate and was known to have antioxidant activity in place of sodium ascorbate. The results are shown in Table 3.

**Table 3**

| | 1 | 2 | 3 | 4 |
|---|---|---|---|---|
| *Escherichia coli* | + | + | + | - |
| *Pseudomonas aeruglnosa* | + | + | + | + |
| *Proteus vulgaris* | + | + | ± | - |
| *Enterobactor cloacae* | + | + | + | - |
| *Bacillus subtillis* | + | + | + | ± |
| *Bacillus cereus* | + | + | ± | - |
| *Lactobacillus casei* | + | + | ± | - |
| *Streptococcus eqinus* | + | + | ± | - |

| | | | | |
|---|---|---|---|---|
| 1 ... Neither sodium erythorbate nor 1,5-D-anhydrofructose was added. | | | | |
| 2 ... 0.2% of sodium erythorbate was added. | | | | |
| 3 ... 1% of 1,5-D-anhydrofructose was added. | | | | |
| 4 ... 0.2% of sodium erythorbate and 1% of 1,5-D-anhydrofructose were added. | | | | |
| +, ± and - represent the following conditions of the colonies. | | | | |
| +: A colony when neither sodium erythorbate nor 1,5-D-anhydrofructose is added and a colony which is as large as the colony when neither sodium erythorbate nor 1,5-D-anhydrofructose is added. | | | | |
| ±: A colony can be detected but its growth is apparently inhibited. | | | | |
| -: A colony cannot be detected. | | | | |

Table 3 shows that sodium erythorbate also has an effect of enhancing the 1,5-D-anhydrofructose's effect of suppressing and inhibiting growth of bacteria. Next, to prove that the effect of enhancing antibacterial activity of 1,5-D-anhydrofructose was not unique to sodium ascorbate and sodium erythorbate and other antioxidants also have the same effect, the same test was carried out by use of butylhydroxyanisol (hereinafter abbreviated as "BHA") and dibutylhydroxytoluene (hereinafter abbreviated as "BHT") as the other antioxidants. Culture media used were the same as those used for testing sodium ascorbate and sodium erythorbate except that they had a pH of 7.0. The results are shown in Table 4. The substances were used at concentrations shown in the legend of Table 4.

**Table 4**

| | 1 | 2 | 3 | 4 | 5 | 6 |
|---|---|---|---|---|---|---|
| *Escherichia coli* | + | + | - | + | - | ± |
| *Pseudomonas aeruginosa* | + | + | + | + | ± | + |
| *Proteus vulgaris* | + | ± | - | ± | - | - |
| *Enterobactor cloacae* | + | + | ± | + | - | + |
| *Bacillus subtillis* | + | + | - | - | - | - |
| *Bacillus* cereus | + | ± | - | - | - | - |
| *Lactobacillus casei* | + | ± | - | - | - | - |
| *Streptococcus eqinus* | + | ± | - | - | - | - |

| | | | | | | |
|---|---|---|---|---|---|---|
| 1 ... None of BHA, BHT and 1,5-D-anhydrofructose was added. | | | | | | |
| 2 ... 1% of 1,5-D-anhydrofructose was added. | | | | | | |
| 3 ... 0.5% of BHA was added. | | | | | | |
| 4 ... 0.5% of BHT was added. | | | | | | |
| 5 ... 0.5% of BHA and 1% of 1, 5-D-anhydrofructose were added. | | | | | | |
| 6 ... 0.5% of BHT and 1% of 1,5-D-anhydrofructose were added. | | | | | | |
| +, ± and - represent the following conditions of the colonies. | | | | | | |
| +: A colony when none of BHA, BHT and 1,5-D-anhydrofructose is added and a colony which is as large as the colony when none of BHA, BHT and 1,5-D-anhydrofructose is added. | | | | | | |
| ±: A colony can be detected but its growth is apparently inhibited. | | | | | | |
| -: A colony cannot be detected. | | | | | | |

0.5% of BHA and 0.5% of BHT independently showed antibacterial activity, and particularly, they exhibited a growth inhibitory effect against all of the examined gram-positive bacteria. Meanwhile, it is found that their effects of inhibiting growth of the gram-negative bacteria are enhanced in the co-presence of 1.0% of 1,5-D-anhydrofructose. 1,5-D-anhydrofructose showed a weak growth suppressing effect against the gram-negative bacteria, and a minimum growth inhibitory concentration of 1,5-D-anhydrofructose for each of the examined bacteria was 3.0% or higher. However, as a result of conducting the above tests, it was found that 1,5-D-anhydrofructose could exhibit the effect even at a low concentration of 1.0% when used in combination with an antioxidant such as sodium ascorbate, sodium erythorbate, BHA or BHT.

Then, influences on growth of a variety of bacteria in the presence of 1,5-D-anhydrofructose with or without sodium citrate, sodium polyphosphate, sodium malate, sodium gluconate or sodium tartrate all of which had a chelate effect were examined. The results are shown in Table 5. Further, Table 5 also shows the results of culturing the bacteria by adding sodium ascorbate or sodium erythorbate both of which had antioxidant activity in addition to the chelating agents and 1,5-D-anhydrofructose. Those cultured in the absence of the chelating agents, 1,5-D-anhydrofructose, sodium ascorbate and sodium erythorbate were taken as controls. Bacterial strains used in the this test are the same as those listed in Table 1. As for an agar medium, a method for inoculating the bacteria, conditions for culturing the bacteria, and a method for evaluating a growth suppressing/inhibiting ability, they are the same as those used in the tests whose results are shown in Tables 2 and 3.

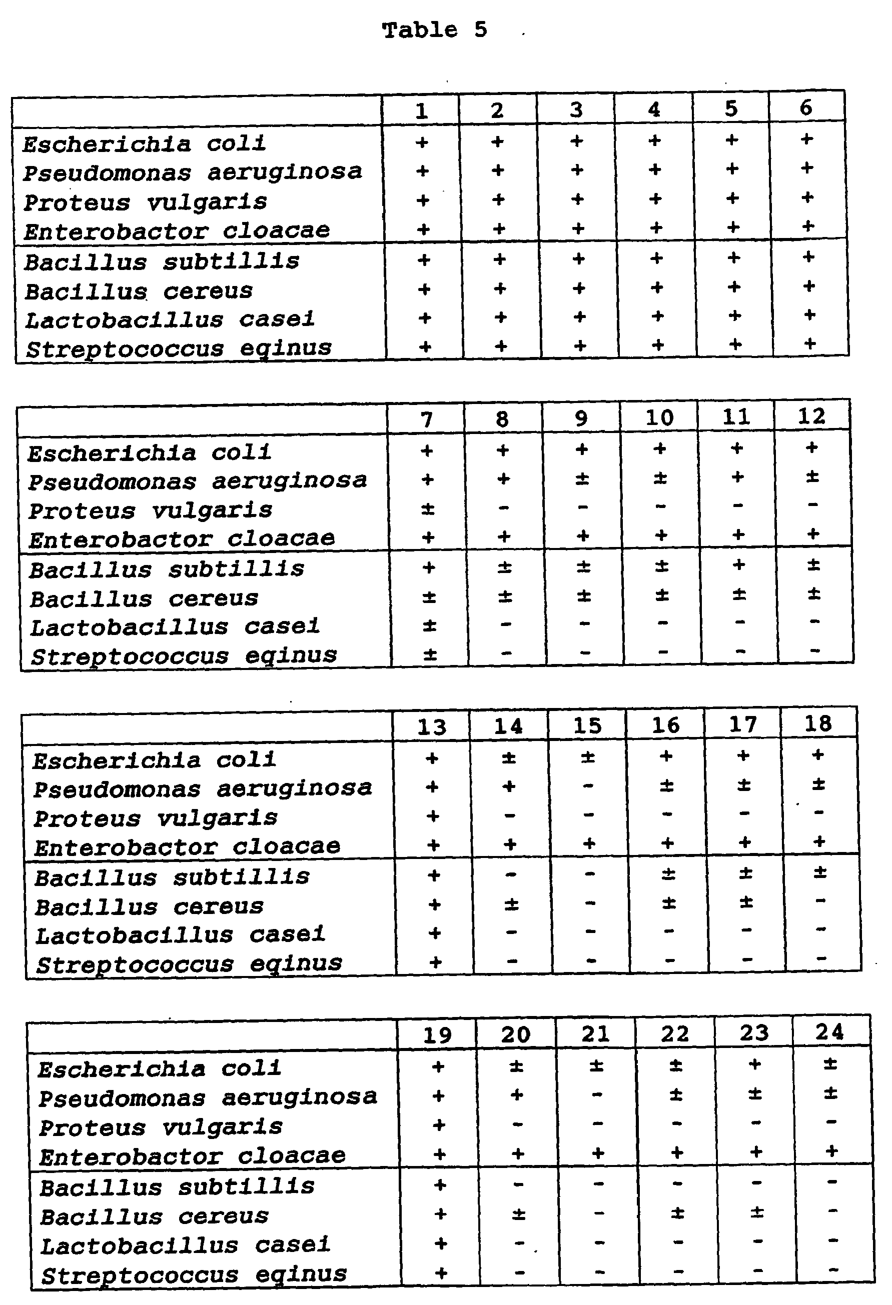

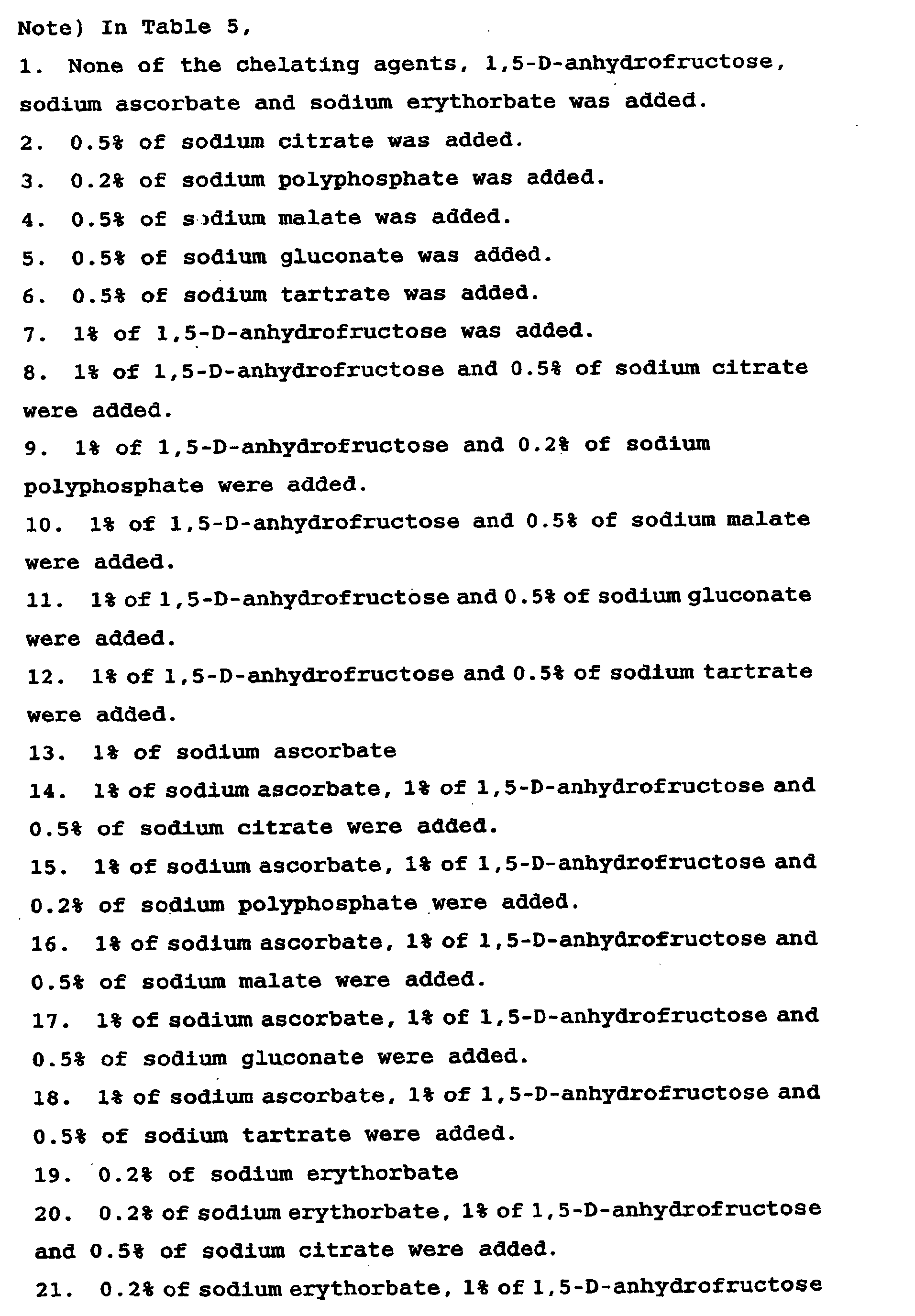

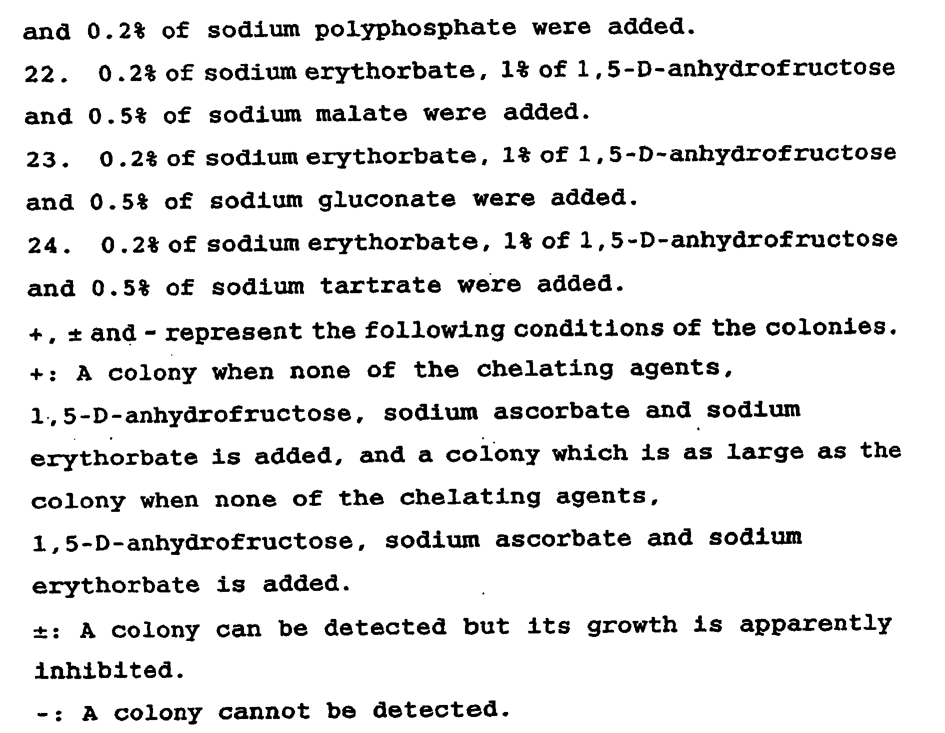

Table 5 shows the following. It is found from comparisons of the collum 1 to 12 that the chelating agents do not have a growth suppressing ability against any of the bacteria when solely used at the concentrations at which they were used in the test and that although 1.0% of 1,5-D-anhydrofructose has some growth suppressing and Inhibiting effect, its growth inhibiting effect is enhanced in the co-presence of the chelating agents. From comparisons of 1, 13 and 19, it is found that sodium ascorbate and sodium erythorbate do not inhibit growth of the bacteria when solely used, just as shown by the results shown in Tables 2 and 3. However, it is found from comparisons of 8 to 12 with 14 to 18 and with 20 to 24 that the effect of suppressing growth of the bacteria by a combination of 1,5-D-anhydrofructose and the chelating agent is further enhanced in the co-presence of sodium ascorbate or sodium erythorbate. Further, the effects of enhancing the effect of the chelating agents, sodium ascorbate and sodium erythorbate on inhibiting growth of the bacteria, cover the gram-negative bacteria as well as the gram-positive bacteria. Since catechin, tocopherol and carotenoid also exhibit the above effect exhibited by sodium ascorbate and sodium erythorbate, it can be said that a wide variety of antioxidant substances can exhibit the same effect. Accordingly, it is understood that the bacteria growth suppressing effect exhibited by 1,5-D-anhydrofructose is enhanced in the co-presence of a chelating agent and further enhanced by addition of an antioxidant.

As the antioxidant in the present invention, any substance having antioxidant activity can be used. Of the substances, as an antioxidant material to be used in food or beverages, an antioxidant which is a food ingredient is preferably used, for example. Typical examples thereof include the following antioxidants, i.e., ascorbic acid and its sodium salt, potassium salt, calcium salt and fatty acid ester; erythorbic acid and its sodium salt, potassium salt, calcium salt and fatty acid ester; α-tocopherol, β-tocopherol, γ-tocopherol, δ-tocopherol, β-carotene, carotenoid, catechin, tannin, flavonoid, anthocyanin, polyphenol, BHA, BHT, uric acid and its salt, docosahexaenoic acid (hereinafter abbreviated as "DHA") and its salt, eicosapentaenoic acid (hereinafter abbreviated as "EPA") and its salt, ethylenediaminetetraacetic acid (hereinafter abbreviated as "EDTA") and its salt, guaiac, isopropyl citrate, nordihydroguaiaretic acid and its salt, and gallic acid ester.

Meanwhile, as the chelating agent in the present invention, any substance having chelate activity can be used. Of the materials, as a chelate material to be used in food or beverages, a chelating agent which is a food ingredient is preferably used, for example. Typical examples thereof include the following chelating agents, i.e., citric acid and its sodium salt, potassium salt and calcium salt; polyphosphoric acid and its sodium salt, potassium salt and calcium salt; malic acid and its sodium salt, potassium salt and calcium salt; gluconic acid and its sodium salt, potassium salt and calcium salt; and tartaric acid and its sodium salt, potassium salt and calcium salt.

Although ratios of the chelating agent and the antioxidant to 1,5-D-anyhydrofructose in the present invention vary depending on types of the chelating agent and the antioxidant, each amounts of the chelating agent and the antioxidant are preferably 0.01 to 100 parts by weight, more preferably 0.1 to 10 parts by weight, per part by weight of 1,5-D-anhydrofructose.

The agent of the present invention may contain an inert carrier and a supplement aid in addition to the chelating agent, the antioxidant and 1,5-D-anhydrofructose.

Typical examples of the inert carrier include saccharides such as starch, maltodextrin, cyclodextrin, roasted dextrin, sugar such as sucrose, glucose, maltose and lactose, viscous polysaccharides such as carboxymethyl cellulose, agar, decomposed agar, carageenan, glucomannan, locust bean gum and xanthan gum, grain flours such as wheat flour, rice flour and corn flour, proteins such as defatted soybeans, non-fat dry milk and a corn protein. Further, when the agent of the present invention is in the form of a liquid or gel, typical examples of the inert carrier further include substances which are liquid at room temperature and atmospheric pressure, such as water and alcohols, in addition to the foregoing substances.

Illustrative examples of the supplement aid include a variety of acids and salts of the acids, such as adipic acid, propionic acid, sorbic acid, succinic acid, benzoic acid, carbonic acid and a nitrite.

The agent of the present invention may take a variety of forms such as a solution, granule, powder, tablet, suspension and gel.

### Examples

Hereinafter, the present invention will be described in more detail with reference to Examples. The present invention shall not be limited by the Examples in any way.

### Example 1 and Comparative Examples 1 to 3 (Test for Keeping Quality by Preservation)

To 1 kg of wheat flour, 30 g of salt and 300 g of water, 15 g of sodium ascorbate and 15 g of 1,5-D-anhydrofructose were added in Example 1, 15 g of 1,5-D-anhydrofructose was added in Comparative Example 1, 15 g of glucose was added in Comparative Example 2, and 15 g of sodium ascorbate and 15 g of glucose were added in Comparative Example 3 so as to produce fresh wheat noodles by use of a roll noodle making machine. The fresh noodles were preserved at room temperature (22 to 30°C), and the aerobic plate count in 1 g of the noodles was determined with time. The results are shown in Table 6.

**Table 6**

| Number of Days Preserved | 0 | 1 | 2 | 3 |
|---|---|---|---|---|
| Ex.1 | 1.1 x 10³ | 2.1 x 10³ | 4.8 x 10⁴ | 3.2 x 10⁶ |
| C.Ex.1 | 8.0 x 10² | 7.2 x 10⁴ | 3.6 x 10⁶ | >10⁷ |
| C.Ex.2 | 2.2 x 10³ | 5.2 x 10⁶ | >10⁷ | - |
| C.Ex.3 | 1.8 x 10³ | 4.4 x 10⁶ | >10⁷ | - |

| | | | | |
|---|---|---|---|---|
| Ex.: Example, C.Ex.: Comparative Example | | | | |

### Example 2 and Comparative Examples 4 to 6 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 10 g of sodium ascorbate and 10 g of 1,5-D-anhydrofructose were added in Example 2, 10 g of 1,5-D-anhydrofructose was added in Comparative Example 4, 10 g of glucose was added in Comparative Example 5, and 10 g of sodium ascorbate and 10 g of glucose were added in Comparative Example 6. After mixed uniformly, the resulting mashed potatoes were preserved at 25° C and a humidity of 80%, and the aerobic plate count in 1 g was determined. The results are shown in Table 7.

**Table 7**

| Number of Hours Preserved (Hour) | 0 | 8 | 16 | 24 | 32 |
|---|---|---|---|---|---|
| Ex.2 | 6.4 x 10² | 1.0 x 10² | 1.8 x 10² | 1.1 x 10³ | 2.3 x 10⁴ |
| C.Ex.4 | 6.5 x 10² | 3.1 x 10² | 6.0 x 10² | 7.0 x 10³ | 9.2 x 10⁴ |
| C.Ex.5 | 6.2 x 10² | 1.6 x 10³ | 5.8 x 10³ | 3.2 x 10⁵ | >10⁷ |
| C.Ex.6 | 5.4 x 10² | 1.1 x 10³ | 7.3 x 10³ | 2.4 x 10⁵ | >10⁷ |

| | | | | | |
|---|---|---|---|---|---|
| Ex.: Example, C.Ex.: Comparative Example | | | | | |

### Example 3 (Test for Keeping Quality by Preservation)

1.0 Liter of solution prepared by adding water to 100 g of sugar, 10 g of brown sugar, 300 g of plum vinegar, 50 g of sea tangle extract powders and 30 g of salt was used as a seasoning solution. To 100 g of sliced cucumbers each having a thickness of 1 cm, 100 ml of the seasoning solution was added, and 2 g of sodium erythorbate and 10 g of 1,5-D-anhydrofructose were further added in Example 3, 10 g of 1,5-D-anhydrofructose was further added in Comparative Example 7, 10 g of glucose was further added in Example 8, and 2 g of sodium erythorbate and 10 g of glucose were further added in Comparative Example 9, and the resulting cucumbers were sealed. Ten packs were prepared for each of Example 3 and Comparative Examples 7, 8 and 9 and preserved at room temperature (22 to 30°C). Thereafter, the number of packs filled with gas was determined with the passage of time. The results are shown in Table 8.

**Table 8**

| Number of Days Preserved | 0 | 1 | 2 | 3 | 4 |
|---|---|---|---|---|---|
| Ex.3 | 0 | 0 | 1 | 3 | 9 |
| C.Ex.7 | 0 | 1 | 2 | 6 | 10 |
| C.Ex.8 | 0 | 3 | 7 | 10 | 10 |
| C.Ex.9 | 0 | 2 | 9 | 10 | 10 |

| | | | | | |
|---|---|---|---|---|---|
| Ex.: Example, C.Ex.: Comparative Example | | | | | |

### Example 4 (Skin Toner)

50 g of glycerine, 40 g of propylene glycol, 20 g of sorbitan monostearate, 100 g of ethanol, 10 g of citric acid and 700 g of purified water were mixed and dissolved. After a pH of the mixture was adjusted to 5.5 with a sodium hydroxide solution, purified water was added to the resulting mixture so as to adjust a volume of the mixture to 1 liter. To the mixture, 10 g of sodium ascorbate and 10 g of 1,5-D-anhydrofructose were added in Example 4, 10 g of 1,5-D-anhydrofructose was added in Comparative Example 10, 10 g of trehalose was added in Comparative Example 11, and 10 g of sodium ascorbate and 10 g of trehalose were added in Comparative Example 12. The resulting mixtures were stirred while it was heated so as to fully dissolve these materials. The mixtures obtained in Example 4 and Comparative Examples 10, 11 and 12 were independently charged into separate sterilized bottles in an amount of 100 ml. The bottles were kept at 25°C, and the number of bacteria contained in 1 ml of the sample was determined with time. The results are shown in Table 9.

**Table 9**

| Number of Days Preserved | 0 | 1 | 2 | 3 | 4 |
|---|---|---|---|---|---|
| Ex.4 | 1.2 x 10 | 1.6 x 10 | 3.2 x 10 | 4.2 x 10 | 8.3 x 10 |
| C.Ex.10 | 1.4 x 10 | 2.1 x 10 | 7.2 x 10 | 2.1 x 10² | 4.3 x 10² |
| C.Ex.11 | 1.1 x 10 | 8.3 x 10 | 3.2 x 10² | 9.6 x 10² | 2.6 x 10³ |
| C.Ex.12 | 1.3 x 10 | 9.1 x 10 | 4.4 x 10² | 1.8 x 10³ | 5.3 x 10³ |

| | | | | | |
|---|---|---|---|---|---|
| Ex.: Example, C.Ex.: Comparative Example | | | | | |

### Example 5 and Comparative Examples 13 to 15 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 5 g of sodium citrate and 10 g of 1,5-D-anhydrofructose were added in Example 5, 10 g of 1,5-D-anhydrofructose was added in Comparative Example 13, 10 g of glucose was added in Comparative Example 14, and 5 g of sodium citrate and 10 g of glucose were added in Comparative Example 15. After stirred uniformly, the resulting mashed potatoes were preserved at 25°C and a humidity of 80%, and the aerobic plate count in 1 g was determined. The results are shown in Table 10.

The following results of Examples 5 to 14 and Comparative Examples 13 to 19 are the results of tests which were performed at the same time.

**Table 10**

| Number of Hours Preserved (Hour) | 0 | 8 | 16 | 24 | 32 |
|---|---|---|---|---|---|
| Ex.5 | 5.8 x 10² | 2.2 x 10 | 5.6 x 10 | 3.1 x 10² | 7.6 x 10³ |
| C.Ex.13 | 5.3 x 10² | 1.2 x 10² | 3.0 x 10² | 6.2 x 10³ | 1.3 x 10⁵ |
| C.Ex.14 | 6.2 x 10² | 2.8 x 10³ | 7.2 x 10³ | 9.2 x 10⁵ | >10⁷ |
| C.Ex.15 | 6.8 x 10² | 2.0 x 10³ | 6.2 x 10³ | 3.3 x 10⁶ | >10⁷ |

| | | | | | |
|---|---|---|---|---|---|
| Ex.: Example, C.Ex.: Comparative Example | | | | | |

### Example 6 and Comparative Example 16 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 2 g of sodium polyphosphate and 10 g of 1,5-D-anhydrofructose were added in Example 6, and 2 g of sodium polyphosphate and 10 g of glucose were added in Comparative Example 16. After stirred uniformly, both were preserved at 25° C and a humidity of 80%, and the aerobic plate count in 1 g was determined. The results are shown in Table 11.

**Table 11**

| Number of Hours Preserved (Hour) | 0 | 8 | 16 | 24 | 32 |
|---|---|---|---|---|---|
| Ex.6 | 6.2 x 10² | 1.8 x 10 | 2.3 x 10 | 1.3 x 10² | 2.2 x 10³ |
| C.Ex.16 | 5.8 x 10² | 1.4 x 10³ | 4.2 x 10³ | 6.3 x 10⁵ | >10⁷ |

| | | | | | |
|---|---|---|---|---|---|
| Ex.: Example, C.Ex.: Comparative Example | | | | | |

### Example 7 and Comparative Example 17 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 5 g of sodium malate and 10 g of 1,5-D-anhydrofructose were added in Example 7, and 5 g of sodium malate and 10 g of glucose were added in Comparative Example 17. After stirred uniformly, both were preserved at 25°C and a humidity of 80%, and the aerobic plate count in 1 g was determined. The results are shown in Table 12.

**Table 12**

| Number of Hours Preserved (Hour) | 0 | 8 | 16 | 24 | 32 |
|---|---|---|---|---|---|
| Ex.7 | 6.4 x 10² | 2.6 x 10 | 4.2 x 10 | 4.2 x 10² | 1.3 x 10⁴ |
| C.Ex.17 | 7.2 x 10² | 3.3 x 10³ | 5.2 x 10³ | 2.1 x 10⁶ | >10⁷ |

| | | | | | |
|---|---|---|---|---|---|
| Ex.: Example, C.Ex.: Comparative Example | | | | | |

### Example 8 and Comparative Example 18 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 5 g of sodium gluconate and 10 g of 1,5-D-anhydrofructose were added in Example 8, and 5 g of sodium gluconate and 10 g of glucose were added in Comparative Example 18. After stirred uniformly, both were preserved at 25°C and a humidity of 80%, and the aerobic plate count in 1 g was determined. The results are shown in Table 13.

**Table 13**

| Number of Hours Preserved (Hour) | 0 | 8 | 16 | 24 | 32 |
|---|---|---|---|---|---|
| Ex.8 | 5.9 x 10² | 2.7 x 10 | 8.2 x 10 | 1.1 x 10³ | 4.3 x 10⁴ |
| C.Ex.18 | 6.4 x 10² | 3.1 x 10³ | 8.3 x 10³ | 5.4 x 10⁵ | >10⁷ |

| | | | | | |
|---|---|---|---|---|---|
| Ex.: Example, C.Ex.: Comparative Example | | | | | |

### Example 9 and Comparative Example 19 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 5 g of sodium tartrate and 10 g of 1,5-D-anhydrofructose were added in Example 9, and 5 g of sodium tartrate and 10 g of glucose were added in Comparative Example 19. After stirred uniformly, both were preserved at 25°C and a humidity of 80%, and the aerobic plate count in 1 g was determined. The results are shown in Table 14.

**Table 14**

| Number of Hours Preserved (Hour) | 0 | 8 | 16 | 24 | 32 |
|---|---|---|---|---|---|
| Ex.9 | 7.2 x 10² | 1.4 x 10 | 2.1 x 10 | 3.3 x 10² | 3.1 x 10³ |
| C.Ex.19 | 5.6 x 10² | 1.4 x 10³ | 7.3 x 10³ | 3.4 x 10⁵ | >10⁷ |

| | | | | | |
|---|---|---|---|---|---|
| Ex.: Example, C.Ex.: Comparative Example | | | | | |

### Example 10 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 10 g of sodium ascorbate, 5 g of sodium citrate and 10 g of 1,5-D-anhydrofructose were added. After stirred uniformly, the resulting mashed potato was preserved at 25° C and a humidity of 80%, and the aerobic plate count in 1 g was determined. The results are shown in Table 15.

**Table 15**

| Number of Hours Preserved (Hour) | 0 | 8 | 16 | 24 | 32 |
|---|---|---|---|---|---|
| Ex.10 | 5.5 x 10² | 5 | 8 | 73 | 5.6 x 10² |

| | | | | | |
|---|---|---|---|---|---|
| Ex.: Example | | | | | |

### Example 11 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 10 g of sodium ascorbate, 2 g of sodium polyphosphate and 10 g of 1,5-D-anhydrofructose were added. After stirred uniformly, the resulting mashed potato was preserved at 25° C and a humidity of 80%, and the aerobic plate count in 1 g was determined. The results are shown in Table 16.

**Table 16**

| Number of Hours Preserved (Hour) | 0 | 8 | 16 | 24 | 32 |
|---|---|---|---|---|---|
| Ex.11 | 6.4 x 10² | 6 | 11 | 57 | 3.6 x 10² |

| | | | | | |
|---|---|---|---|---|---|
| Ex.: Example | | | | | |

### Example 12 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 2 g of sodium erythorbate, 5 g of sodium malate and 10 g of 1,5-D-anhydrofructose were added. After stirred uniformly, the resulting mashed potato was preserved at 25° C and a humidity of 80%, and the aerobic plate count in 1 g was determined. The results are shown in Table 17.

**Table 17**

| Number of Hours Preserved (Hour) | 0 | 8 | 16 | 24 | 32 |
|---|---|---|---|---|---|
| Ex.12 | 6.4 x 10² | 22 | 15 | 32 | 2.1 x 10² |

| | | | | | |
|---|---|---|---|---|---|
| Ex.: Example | | | | | |

### Example 13 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 2 g of BHT, 5 g of sodium tartrate and 10 g of 1,5-D-anhydrofructose were added. After stirred uniformly, the resulting mashed potato was preserved at 25°C and a humidity of 80%, and the aerobic plate count in 1 g was determined. The results are shown in Table 18.

**Table 18**

| Number of Hours Preserved (Hour) | 0 | 8 | 16 | 24 | 32 |
|---|---|---|---|---|---|
| Ex.13 | 6.4 x 10² | 0 | 3 | 5 | 33 |

| | | | | | |
|---|---|---|---|---|---|
| Ex.: Example | | | | | |

### Example 14 (Test for Keeping Quality by Preservation)

1 kg of boiled potatoes were peeled and smashed with a wood stick so as to prepare a mashed potato. In addition to 10 g of salt, 5 g of pepper, 50 g of mayonnaise and 100 ml of milk, 1 g of green tea polyphenol, 5 g of sodium citrate and 10 g of 1,5-D-anhydrofructose were added. After stirred uniformly, the resulting mashed potato was preserved at 25° C and a humidity of 80%, and the aerobic plate count in 1 g was determined. The results are shown in Table 19.

**Table 19**

| Number of Hours Preserved (Hour) | 0 | 8 | 16 | 24 | 32 |
|---|---|---|---|---|---|
| Ex.14 | 6.4 x 10² | 2 | 5 | 22 | 92 |

| | | | | | |
|---|---|---|---|---|---|
| Ex.: Example | | | | | |

### Example 15 (Fruit Juice Beverage)

To Bx12 prepared by adding sterilized distilled water to concentrated grape juice, 1.0% of 1,5-D-anhydrofructose and 1.0% of sodium ascorbate were added in Example 15, 1.0% of 1,5-D-anhydrofructose was added in Comparative Example 20, and 1.0% of sodium ascorbate was added in Comparative Example 21. After stirred uniformly, the mixtures were preserved at 25° C and a humidity of 80%, and the aerobic plate count in 1 g was determined. The results are shown in Table 20.

**Table 20**

| Number of Hours Preserved (Hour) | 0 | 8 | 16 | 24 | 32 |
|---|---|---|---|---|---|
| Ex.15 | 58 | 1.2 x 10² | 2.6 x 10² | 4.2 x 10² | 3.3 x 10³ |
| C.Ex.20 | 30 | 2.1 x 10² | 7.2 x 10² | 4.2 x 10³ | 6.1 x 10⁴ |
| C.Ex.21 | 64 | 1.8 x 10³ | 4.2 x 10⁴ | 3.6 x 10⁵ | >10⁷ |

| | | | | | |
|---|---|---|---|---|---|
| Ex.: Example, C.Ex.: Comparative Example | | | | | |

### Example 16 (Liquid Preparation)

A liquid preparation comprising 15 parts of sodium ascorbate, 50 parts of 1,5-D-anhydrofructose, 10 parts of glucose, 25 parts of water, a very small amount of salt and other components and having a pH of 5.0 was prepared according to a method known per se.

### Example 17 (Powdery Preparation)

The liquid preparation of Example 16 was freeze-dried by a method known per se so as to prepare a powdery preparation.

### Example 18 (Powdery Preparation)

The liquid preparation of Example 16 was spray-dried by a method known per se so as to prepare a powdery preparation.

### Example 19 (Granular Preparation)

The powdery preparation of Example 18 was granulated by a method known per se so as to prepare a granular preparation which is easily dissolved in water.

### Example 20 (Liquid Preparation)

A liquid preparation comprising 14 parts of sodium ascorbate, 14 parts of 1,5-D-anhydrofructose, 7 parts of glucose, 65 parts of water, a very small amount of salt and other components and having a pH of 5.0 was prepared in the same manner as in Example 16.

### Example 21 (Liquid Preparation)

A liquid preparation comprising 14 parts of sodium ascorbate, 14 parts of 1,5-D-anhydrofructose, 7 parts of glucose, 3 parts of acetic acid, 62 parts of water, a very small amount of salt and other components and having a pH of 5.0 was prepared in the same manner as in Example 16.

### Example 22 (Powdery Preparation)

The liquid preparation of Example 20 was freeze-dried by a method known per se so as to prepare a powdery preparation.

### Example 23 (Powdery Preparation)

The liquid preparation of Example 20 was concentrated and then spray-dried by a method known per se so as to prepare a powdery preparation.

### Example 24 (Granular Preparation)

The powdery preparation of Example 23 was granulated by a method known per se so as to prepare a granular preparation which is easily dissolved in water.

## Claims

1. An agent for suppressing or inhibiting growth of bacteria, the agent comprising:
(A) 1,5-D-anhydrofructose, and
(B) at least one antioxidant or chelating agent.

2. An agent according to claim 1 wherein (B) comprises at least one antioxidant and at least one chelating agent.

3. An agent according to claim 1 or claim 2 comprising an antioxidant which is a food ingredient.

4. An agent according to any one of the preceding claims comprising a chelating agent which is a food ingredient.

5. An agent according to any preceding claim comprising as antioxidant, at least one compound selected from ascorbic acid and its sodium, potassium and calcium salts and its fatty acid esters, erythorbic acid and its sodium, potassium and calcium salts and its fatty acid esters, α-tocopherol, β-tocopherol, γ-tocopherol, δ-tocopherol, β-carotene, carotenoid, catechin, tannin, flavonoid, anthocyanin, polyphenol, BHA, BHT, uric acid and its salts, DHA and its salts, EPA and its salts, EDTA and its salts, guaiac, isopropyl citrate, nordihydroguaiaretic acid and its salts, and gallic acid esters.

6. An agent according to any preceding claim comprising, as chelating agent, at least one compound selected from citric acid, polyphosphoric acid, malic acid, gluconic acid, tartaric acid, and their sodium, potassium and calcium salts.

7. Use of 1, 5-D-anhydrofrutose for suppressing or inhibiting growth of bacteria in the co-presence of at least one antioxidant or chelating agent.

8. Use according to claim 7 in the co-presence of at least one antioxidant and at least one chelating agent.

9. Use according to claim 7 or 8 wherein the antioxidant is a food ingredient.

10. Use according to any one of claims 7 to 9 wherein the chelating agent is a food ingredient.

11. Use according to any one of claims 7 to 10 wherein the antioxidant is at least one compound selected from ascorbic acid and its sodium, potassium and calcium salts and its fatty acid esters, erythorbic acid and its sodium, potassium and calcium salts and its fatty acid esters, α-tocopherol, β-tocopherol, γ-tocopherol, δ-tocopherol, β-carotene, carotenoid, catechin, tannin, flavonoid, anthocyanin, polyphenol, BHA, BHT, uric acid and its salts, DHA and its salts, EPA and its salts, EDTA and its salts, guaiac, isopropyl citrate, nordihydroguaiaretic acid and its salts, and gallic acid esters.

12. Use according to any one of claims 7 to 11 wherein the chelating agent is at least one compound selected from citric acid, polyphosphoric acid, malic acid, gluconic acid, tartaric acid, and their sodium, potassium and calcium salts.

## Patentansprüche

1. Mittel zur Unterdrückung oder Inhibierung des Wachstums von Bakterien, wobei das Mittel Folgendes umfasst:
(A) 1,5-D-Anydrofructose und
(B) mindestens ein Antioxidans oder Komplexbildner.

2. Mittel gemäß Anspruch 1, wobei (B) mindestens ein Antioxidans und mindestens einen Komplexbildner umfasst.

3. Mittel gemäß Anspruch 1 oder Anspruch 2, umfassend ein Antioxidans, welches ein Nahrungsbestandteil ist.

4. Mittel gemäß mindestens einem der vorhergehenden Ansprüche, umfassend einen Komplexbildner, welcher ein Nahrungsmittelbestandteil ist.

5. Mittel gemäß mindestens einem der vorhergehenden Ansprüche, umfassend als Antioxidans mindestens eine Verbindung, gewählt aus Ascorbinsäure und deren Natrium-, Kalium- und Calciumsalzen und deren Fettsäureestern, Erythorbinsäure und deren Natrium-, Kalium- und Calciumsalzen und deren Fettsäureestern, α-Tocopherol, β-Tocopherol, γ-Tocopherol, δ-Tocopherol, β-Karotin, Karotenoid, Katechin, Tannin, Flavonoid, Anthocyanin, Polyphenol, BHA, BHT, Harnsäure und deren Salzen, DHA und deren Salzen, EPA und deren Salzen, EDTA und deren Salzen, Guajak bzw. Franzosenholz, Isopropylcitrat, Nordihydroguajaretsäure und deren Salzen und Gallussäureestern.

6. Mittel gemäß mindestens einem der vorhergehenden Ansprüche, umfassend als Komplexbildner mindestens eine Verbindung, gewählt aus Zitronensäure, Polyphosphorsäure, Äpfelsäure, Gluconsäure, Weinsäure und deren Natrium-, Kalium- und Calciumsalzen.

7. Verwendung von 1,5-D-Anhydrofructose zur Unterdrückung oder Inhibierung des Wachstums von Bakterien bei gleichzeitiger Anwesenheit von mindestens einem Antioxidans oder Komplexbildner.

8. Verwendung nach Anspruch 7 bei gleichzeitiger Anwesenheit von mindestens einem Antioxidans und mindestens einem Komplexbildner.

9. Verwendung gemäß Anspruch 7 oder 8, wobei das Antioxidans ein Nahrungsmittelbestandteil ist.

10. Verwendung gemäß mindestens einem der Ansprüche 7 bis 9, wobei der Komplexbildner ein Nahrungsmittelbestandteil ist.

11. Verwendung gemäß mindestens einem der Ansprüche 7 bis 10, wobei das Antioxidans mindestens eine Verbindung ist, gewählt aus Ascorbinsäure und deren Natrium-, Kalium- und Calciumsalzen und deren Fettsäureestern, Erythorbinsäure und deren Natrium-, Kalium- und Calciumsalzen und deren Fettsäureestern, α-Tocopherol, β-Tocopherol, γ-Tocopherol, δ-Tocopherol, β-Karotin, Karotenoid, Katechin, Tannin, Flavonoid, Anthocyanin, Polyphenol, BHA, BHT, Harnsäure und deren Salzen, DHA und deren Salzen, EPA und deren Salzen, EDTA und deren Salzen, Guajak, Isopropylcitrat, Nordihydroguajaretsäure und deren Salzen und Gallussäureestern.

12. Verwendung gemäß mindestens einem der Ansprüche 7 bis 11, wobei der Komplexbildner mindestens eine Verbindung ist, gewählt aus Zitronensäure, Polyphosphorsäure, Äpfelsäure, Gluconsäure, Weinsäure und deren Natrium-, Kalium- und Calciumsalzen.

## Revendications

1. Agent pour supprimer ou inhiber la croissance bactérienne, l'agent comprenant :
(A) du 1,5-D-anhydrofructose, et
(B) au moins un antioxydant ou un agent chélateur.

2. Agent selon la revendication 1, dans lequel (B) comprend au moins un antioxydant et au moins un agent chélateur.

3. Agent selon la revendication 1 ou la revendication 2, comprenant un antioxydant qui est un ingrédient alimentaire.

4. Agent selon l'une quelconque des revendications précédentes, comprenant un agent chélateur qui est un ingrédient alimentaire.

5. Agent selon l'une quelconque des revendications précédentes comprenant, en tant qu'antioxydant, au moins un composé choisi parmi l'acide ascorbique et ses sels de sodium, de potassium et de calcium et ses esters d'acide gras, l'acide érythorbique et ses sels de sodium, de potassium et de calcium et ses esters d'acide gras, l'α-tocophérol, le β-tocophérol, le γ-tocophérol, le δ-tocophérol, le β-carotène, le caroténoïde, la catéchine, le tanin, le flavonoïde, l'anthocyanine, le polyphénol, le BHA, le BHT, l'acide urique et ses sels, le DHA et ses sels, l'EPA et ses sels, l'EDTA et ses sels, la résine de gaïac, le citrate d'isopropyle, l'acide nordihydroguaïarétique et ses sels, et les esters de l'acide gallique.

6. Agent selon l'une quelconque des revendications précédentes comprenant, en tant qu'agent chélateur, au moins un composé choisi parmi l'acide citrique, l'acide polyphosphorique, l'acide malique, l'acide gluconique, l'acide tartrique, et leurs sels de sodium, de potassium et de calcium.

7. Utilisation de 1,5-D-anhydrofructose pour la suppression ou l'inhibition de la croissance bactérienne en coprésence d'au moins un antioxydant ou un agent chélateur.

8. Utilisation selon la revendication 7, en coprésence d'au moins un antioxydant et d'au moins un agent chélateur.

9. Utilisation selon la revendication 7 ou 8, dans laquelle l'antioxydant est un ingrédient alimentaire.

10. Utilisation selon l'une quelconque des revendications 7 à 9, dans laquelle l'agent chélateur est un ingrédient alimentaire.

11. Utilisation selon l'une quelconque des revendications 7 à 10, dans laquelle l'antioxydant est au moins un composé choisi parmi l'acide ascorbique et ses sels de sodium, de potassium et de calcium et ses esters d'acide gras, l'acide érythorbique et ses sels de sodium, de potassium et de calcium et ses esters d'acide gras, l'α-tocophérol, le β-tocophérol, le γ-tocophérol, le δ-tocophérol, le β-carotène, le caroténoïde, la catéchine, le tanin, le flavonoïde, l'anthocyanine, le polyphénol, le BHA, le BHT, l'acide urique et ses sels, le DHA et ses sels, l'EPA et ses sels, l'EDTA et ses sels, la résine de gaïac, le citrate d'isopropyle, l'acide nordihydroguaïarétique et ses sels, et les esters de l'acide gallique.

12. Utilisation selon l'une quelconque des revendications 7 à 11, dans laquelle l'agent chélateur est au moins un composé choisi parmi l'acide citrique, l'acide polyphosphorique, l'acide malique, l'acide gluconique, l'acide tartrique, et leurs sels de sodium, de potassium et de calcium.
